Europäisches Patentamt

European Patent Office

Office européen des brevets

(11) Publication number: **0 479 419 A1**

# EUROPEAN PATENT APPLICATION

(21) Application number: **91306977.9**

(22) Date of filing: **30.07.91**

(51) Int. Cl.5: **C07C 227/40**, C07B 63/02, G21F 9/06, C02F 5/12, C07C 229/16

(30) Priority: **01.10.90**
**09.07.91**

(43) Date of publication of application:
**08.04.92 Bulletin 92/15**

(84) Designated Contracting States:
**AT BE CH DE DK ES FR GB GR IT LI LU NL SE**

(71) Applicant: **CHEMICAL WASTE MANAGEMENT, INC.**
**3001 Butterfield Road**
**Oak Brook Illinois 60521(US)**

(72) Inventor: **Halpern, Yuval**
**9514 La Vergne Avenue**
**Skokie, Illinois 60077(US)**

(74) Representative: **Cockbain, Julian et al**
**Frank B. Dehn & Co. Imperial House 15-19, Kingsway**
**London WC2B 6UZ(GB)**

(54) **Process for removing trace metals.**

(57) There is described a process for removing metal salts from a chelating agent (e.g. EDTA) by esterification to produce a solid metal salt and an ester of the chelating agent and by thereafter separating the solid metal salt from the reaction mixture.

EP 0 479 419 A1

The invention relates to a process for removing trace metals, in particular to a process for removing trace metals from aminopolycarboxylic acid (APCA) chelating agents such as EDTA and DTPA, and to APCA peresters produced thereby.

The process of the invention is applicable generally to APCAs and APCA chelates but is most appropriately used in conjunction with the APCA chelating agents that are used on an industrial scale as scavengers and cleaning agents, in particular EDTA and in much of the discussion below reference is made to EDTA as the preferred example of the APCA chelating agents.

EDTA (ethylene diamine tetraacetic acid) is used as a chelating agent for cleaning scale from steam generators used for pressurized water reactors. These steam generators accumulate great quantities of precipitated iron oxides that reduce the heat transfer coefficient and the efficiency of the steam generator. Periodically, it is necessary to clean the precipitated material from these steam generators.

A 10-15% ammonium EDTA salt solution is typically injected into the steam generator. Since EDTA is a powerful chelating agent, it coordinates the iron and solubilizes it. In nuclear power plants, the shell side or secondary side of these steam generators is supposed to be the "clean" side. However, minor leaks in the tubes may occur allowing material from the primary side to leak low levels of radioactivity to the "clean" side, primarily in the form of radioisotopes of cesium, cobalt and other metals.

Acidifying the metal salt containing EDTA chelating solution precipitates metal-salt-contaminated $H_4EDTA$. The cost of stabilization, transportation and disposal of these wastes is high, making reduction of the volume of the metal salt containing $H_4EDTA$ precipitate a cost-effective alternative.

The reduction of the volume of radioactive waste products resulting from steam generator cleaning has been the subject of various US Patents. However, none of the Patents discloses or anticipates a process for eliminating radioactive salts from an EDTA acid precipitate.

US Patent 3669631 to Bellbrook et al. discloses a process for removing radioactive materials from ion-exchange resins. The ion-exchange resins are used to remove radioactive metal isotopes from a cleaning solution. The radioactive isotopes are recovered by dissolving the ion-exchange resins in an acid/catalyst mixture and thereafter recovering the radioactive isotopes from the solution.

US Patent 3791981 to Calmon discloses a method for reducing the volume of radioactive contaminated ion-exchange resins prior to their disposal. The method consists of contacting the ion-exchange resins with an organic solvent to physically reduce the ion-exchange resin volume.

US Patent 4704235 to Arvesen discloses a steam generator descaling method that is accomplished in a single step in the absence of EDTA.

US Patent 4705573 to Wood et al. discloses a descaling process that does not use EDTA. The process comprises contacting a scale-containing surface with a specific reagent. No mention of subsequent reagent purification is made.

An article by Robert W Hay et al. entitled "Reactions of Co-ordinated Ligands. Hydrolysis of Tetramethyl Ethylenediaminetetra-acetate and its Copper (II) Complexes" discloses methods of producing $Me_4EDTA$ from $H_4EDTA$.

A principal object of this invention is to provide a process for removing metal contained in an $H_4EDTA$ complex. The process enables one to achieve a significant reduction in the volume of the unwanted metal containing $H_4EDTA$ complex prior to disposal.

Thus viewed from one aspect the invention provides a process for removing metal from an aminopolycarboxylic acid, said process comprising esterifying said aminopolycarboxylic acid and separating at least some of said metal from the resulting ester.

The process of the invention is preferably effected to yield an APCA ester which is liquid or at least remains in solution in the reaction mixture under conditions where the fully protonated APCA (e.g. $H_4EDTA$) is in the solid phase. Formation of aliphatic or aromatic esters, in particular peresters (i.e. esters in which all carboxy groups are esterified) is preferred and such peresters, which can readily be recycled for re-use by hydrolysis, form a further aspect of the invention. Viewed from this aspect the invention thus provides APCA peresters, especially EDTA-homo- and hetero-tetraesters (with the exception of EDTA-tetramethylester), and in particular esters in which in each ester grouping $COOR_1$ the $R_1$ moiety which is aromatic or aliphatic contains at least 2 carbons, especially 3-10 carbons.

Thus in one broad embodiment the present invention is a process for removing metal contained in a metal contaminated $H_4EDTA$. The metal containing $H_4EDTA$ preferably undergoes esterification in the presence of an esterification reagent to produce an esterification mixture comprising a metal salt and a liquid EDTA ester. The metal salt may then be separated from the esterification mixture.

In a preferred embodiment, the process of the invention may be used for removing radioactive metal from an $H_4EDTA$ precipitate. The radioactive metal may be removed by esterifying a radioactive metal containing $H_4EDTA$, e.g. $H_4EDTA$ containing a radioactive metal salt, with an esterification reagent, conveniently in the presence of an organic solvent, at esterification conditions including a temperature of from about 0°C up to about the boiling point of the esterification reagent or the reaction mixture but no greater than 220°C for a period of time ranging from 15 minutes to about 24 hours or more to provide an esterification mixture comprising a solid radioactive metal salt, excess organic solvent and a liquid esterified EDTA. The liquid esterified EDTA is then recovered from the mixture.

$H_4$EDTA is a white crystalline solid that does not melt but decomposes at 220° Celsius. In addition, $H_4$EDTA is almost insoluble in water, acids or organic liquids. Metal containing $H_4$EDTA precipitates are produced from EDTA salts used as chelating agents. EDTA salt solutions may be used as chelating agents to scavenge entrapped metals such as metals from ion-exchange resins or from scale built up in heat exchangers and similar equipment. EDTA chelating agents are primarily useful in the nuclear power industry for removing materials such as isotopes of plutonium, uranium, thorium, actinium, cesium, cobalt, or other heavy-element isotopes that are contained in scale built up on equipment or that have been entrapped by ion-exchange resins.

EDTA salt solutions, contaminated with metals such as radioactive metals are typically acidified to convert the contaminated EDTA into a contaminated $H_4$EDTA precipitate and to convert the metal into a metal salt. The solid metal salts cannot be removed from the $H_4$EDTA precipitate by applying physical processes such as extraction or fractional crystallization. Typically, the solid metal salt containing $H_4$EDTA is disposed of in a landfill. However, it has now been discovered that the solid metal salt can be removed and recovered from the $H_4$EDTA precipitate by subjecting the metal salt containing $H_4$EDTA precipitate to the esterification process of this invention. The EDTA ester produced by the esterification step can thereafter be disposed of by incineration or landfill, or the EDTA ester can be hydrolyzed to return it once again into $H_4$EDTA or into an EDTA salt.

The metal salts that are entrapped by the $H_4$EDTA may be any metal salt. Where the metal salt is insoluble in the esterification reaction solution it may readily be separated and recovered from said solution. The process of this invention is most useful for removing radioactive metal salts trapped in an $H_4$EDTA precipitate. Such salts might, for example, contain isotopes of radioactive metals such as cobalt, cesium, cerium, plutonium, indium or salts of any radioactive metal isotopes. It is most probable that the radioactive metal salt will be a salt of cesium or cobalt.

According to the process of this invention, the metal containing $H_4$EDTA undergoes esterification in order to remove the metal therefrom. Generally, the purpose of the esterification reaction is to create an EDTA ester that enters the liquid phase (e.g. one that is soluble in an organic solvent used in the esterification mixture or soluble in the excess of the esterifying reagent) while the metal remains in (or is returned to) the solid phase, e.g. where the metal remains in the form of a salt which is insoluble in the esterification mixture. After the metal salt containing $H_4$EDTA precipitate has been esterified, the insoluble solid metal salt can then be recovered from the solution.

The solid metal salt containing $H_4$EDTA precipitate is suitably esterified in the presence of an esterification reagent. The esterification reagent is conveniently any liquid reagent or mixture of liquid reagents capable of converting the $H_4$EDTA precipitate into an EDTA ester. Preferred esterification reagents are alcohols. It is further preferred that the alcohols are primary alcohols such as methanol, ethanol, propanol, butanol and similar primary alcohols and mixtures thereof. The esterification of $H_4$EDTA, an organic acid, with an alcohol such as methanol produces an EDTA ester following the formula below:

$(HOOCCH_2)_2NCH_2CH_2N(CH_2COOH)_2$ + $R_1OH$ + $R_2OH$ + $R_3OH$ + $R_4OH$ ------) $(R_1OOCCH_2)$-$(R_2OOCCH_2)NCH_2CH_2N(CH_2COOR_3)(CH_2COOR_4)$ + $4H_2O$

where $R_1$, $R_2$, $R_3$, and $R_4$, which may be the same or different, are aromatic or aliphatic radicals. The actual identity of $R_1$, $R_2$, $R_3$ and $R_4$ will depend upon the alcohols used as the esterification reagent. The esterification reagent used can include an alcohol or alcohols having 1 or more carbon atoms. However, it is preferred that an esterification reagent is chosen so that $R_1$, $R_2$, $R_3$ and $R_4$ are the same aliphatic radical or a mixture of aliphatic radicals having 2 or more carbon atoms, the same aromatic radical or mixture of aromatic radicals having of 6 or more carbon atoms or a mixture thereof. Finally, it is preferred that $R_1$, $R_2$, $R_3$ and $R_4$ are aliphatic radicals, aromatic radicals, or a mixture thereof, each having from 3 to 10 carbon atoms.

From the formula for the EDTA ester above, it can be seen that the esterification reaction between an organic acid and a primary alcohol produces water. The esterification reaction is typically promoted by removing the water from the reaction mixture by any method available that does not interfere with the esterification reaction. Such methods might include distillation where the esterification reagent has a higher boiling point than the water, chemical means such as a solid desiccant added to the esterification mixture, or other means such as the addition of liquids that complex with the water or by performing the reaction in the presence of a catalyst.

When butanol is used as the esterification reagent, the esterification reaction product is tetrabutyl ethylenediaminetetraacetate or tetrabutyl-EDTA. Tetrabutyl-EDTA is believed to be novel and has the chemical formula:

$$[-CH_2-N(CH_2COOC_4H_9)_2]_2$$

Tetrabutyl-EDTA is produced by combining a butyl alcohol such as n-butanol with an acidic solution containing $H_4$EDTA. The n-butanol combines with the $H_4$EDTA to produce tetrabutyl-EDTA.

The esterification reaction may occur in the presence of an organic solvent. The organic solvent may be the same as the esterification reagent. The purpose of the organic solvent is to stabilize the esterification reaction conditions such as temperature and to promote the contact of the esterification reagent with the metal salt containing $H_4$EDTA precipitate. Additionally, the solvent may dilute the effect of water on the esterification reaction rate. The organic solvent may be polar or non-polar. It is also preferred that the organic solvent be non-protic. By non-protic, it is meant that the organic solvent has no protons to donate to a chemical reaction. It is preferred that the organic solvent is methanol.

The conditions at which the esterification reaction can occur will depend somewhat upon the choice of esterification reagent and the optional organic solvent. Temperature typically promotes the esterification reaction. Therefore, the higher the temperature at which the esterification occurs, the speedier the reaction rate. However, there are some inherent limitations to the esterification of $H_4$EDTA. As mentioned above, $H_4$EDTA begins to decompose at 220°C at atmospheric pressure. Therefore 220°C is generally anticipated to be the maximum temperature at which this esterification reaction can occur. Furthermore, it is likely that the chosen esterification reagent or the organic solvent will boil at a temperature below 220°C. Therefore, the esterification reaction may occur at a temperature up to about 220°C but at or below the boiling point of the organic solvent and/or the esterification reagent chosen. The pressure of the system can be adjusted to increase the temperature at which the esterification reagent or the organic solvent will boil, thus allowing an esterification reaction to occur at a higher temperature than would be allowed if the reaction were occurring at atmospheric pressure.

Using the preferred organic solvent methanol and the preferred esterification reagent methanol, the esterification reaction can occur at a temperature from about 0°C up to the temperature of the boiling point of methanol. The pressure at which the esterification reaction occurs may be modified from about atmospheric pressure up to any pressure necessary to raise the reaction temperature to about 220°C, at which point the $H_4$EDTA precipitate may begin to decompose. The reaction will typically take from about 15 minutes or longer to be complete. It is preferred that the reaction is effected for about 15 minutes to about 24 hours.

The esterification reaction may occur in the presence of an esterification catalyst. Compounds that catalyze the esterification reaction include strong acids, tin salts, organotitanates, silica gel, and cation-exchange resins. It is preferred that the esterification catalyst is a strong acid such as sulfuric acid.

As previously mentioned, the solid metal salt containing $H_4$EDTA precipitate is preferably esterified in the presence of methanol used as both the esterification reagent and as the esterification solvent. The esterification reaction occurs at a pressure ranging from atmospheric pressure up to a pressure at which the boiling point of methanol is about 220°C. The temperature at which the esterification reaction takes place can be any temperature that does not exceed the boiling point of methanol. A preferred temperature range is from about 0°C up to about the boiling point temperature of methanol at the particular pressure chosen.

The reaction mixture produced by the esterification reaction typically comprises metal salts that are insoluble in the reaction mixture, organic solvent, and an EDTA ester. The reaction mixture may also contain unreacted esterification reagent and unreacted $H_4$EDTA precipitate. While it is preferred that insoluble metal salts are the only solid materials remaining in the reaction mixture, it is possible that some of the $H_4$EDTA precipitate material will not become esterified. Even in this situation, the volume of $H_4$EDTA precipitate containing metal salts will have been reduced by the esterification process. The disposal of the solid material will still be less problematic due to the reduction in volume of the material.

The esterification reaction mixture now undergoes a solid-liquid separation step. The purpose of the solid-liquid separation step is to remove the metal salt containing solids material in the reaction mixture from the reaction mixture liquids. The solid-liquid separation may be accomplished by any means known in the art for separating a solid material from a liquid material such as, for example, mechanical separation means or distillation. It is preferred that a mechanical separation means be employed to effect the solid-liquid separation. Such a mechanical separation means might be for example filtration, settling following by decantation of the clarified liquid, or centrifugation. The act of separating the solids, including the solid metal salt, from the esterification reaction mixture produces a separated solid material containing solid metal salts and a clarified esterification mixture. At this point, the recovered solids, including solid metal salts, can be disposed of by landfill methods or other means. The clarified esterification mixture may then be subjected to a number of further processing steps or, alternatively, the clarified esterification solution may be disposed of by incineration or other means.

One processing option is to hydrolyze the clarified esterification mixture. Hydrolysis of the clarified esterification mixture essentially reverses the esterification reaction and converts the EDTA ester into $H_4$EDTA or into EDTA salt. The hydrolysis will occur at hydrolysis conditions in the presence of an acidic or basic aqueous solution. As a result of the reaction of the clarified esterification mixture with water, a crude $H_4$EDTA precipitate and a hydrolysis solution comprising organic solvent and water is produced. When an acidic hydrolysis solution is used, then the EDTA ester is hydrolyzed to $H_4$EDTA precipitate. When a basic solution is used to hydrolyze the EDTA ester, then an EDTA aqueous salt is produced. When an $H_4$EDTA precipitate is produced, it can be separated from the liquid by filtration, after which remaining solvent can be separated from the water by distillation and recovered. The filtered $H_4$EDTA precipitate can then either be incinerated or recycled as a chelating agent for scale removal.

Optionally, the clarified esterification mixture comprising a reagent, a solvent, and EDTA ester may be subjected to liquid separation steps to recover essentially pure component streams of the clarified esterification solution. Such liquid separation steps might include, for example, distillation, adsorption or extraction. It is preferred that distillation be used to recover essentially pure fractions of the organic solvent, the esterification reagent and the EDTA ester.

The recovered organic solvent and esterification reagent can typically be recycled to the esterification step. The EDTA ester can be incinerated, or alternatively it can be subjected to hydrolysis conditions to produce a solid $H_4$EDTA precipitate or an EDTA salt. The hydrolysis of the EDTA ester fraction also produces a small amount of reagent. The EDTA salt can be separated from the hydrolysis solution containing the esterification reagent and either recycled as a chelating agent or incinerated.

As mentioned above, the esterification reagent of this process is preferably methanol. Additionally, the organic solvent of this process is preferably methanol. In this case, the methanol that is not reacted with the $H_4$EDTA precipitate would be separated from the EDTA ester by distillation and recycled back to the $H_4$EDTA esterification step. The EDTA ester then undergoes incineration or hydrolysis, as discussed above.

Certain preferred non-limiting examples of the invention will be discussed below. However, many other examples would also fall within the scope of the present invention.

Examples

Example 1

In this example, $H_4$EDTA contaminated with 170 ppm of nickel was subjected to trace metal cleaning. The nickel contaminated $H_4$EDTA was combined with concentrated $H_2SO_4$ (1:10 molar ratio) and refluxed in an excess of n-butanol. Water, a reaction product, was removed from the reaction solution as an azeotrope with butanol. When the theoretical amount of water was collected, the hydrolysis was deemed complete and the reflux discontinued. A clear solution remained which was cooled to ambient temperature. The clear solution was washed with water and neutralized with a base followed by evaporation at ambient temperature. The clear liquid product was identified by $^1$H and $^{13}$C-NMR as tetrabutyl-Ethylenediaminetetraacetate. The clear solution had a nickel concentration of only 8.36 ppm.

Example 2

In this example, the tetrabutyl-Ethylenediaminetetraacetate of Example 1 was hydrolyzed and converted into $H_4$EDTA. The tetrabutyl-Ethylenediaminetetraacetate of Example 1 was combined with NaOH at a 1:10 molar ratio with an excess of water and refluxed gently while stirring for about fifteen hours. The pH of the solution was made acidic and the white solid $H_4$EDTA produced by acidifying the solution was collected by filtration and dried in a vacuum at 70°C to a constant weight. The nickel content of the solid $H_4$EDTA was found to be 0.92 ppm.

Example 3

A procedure essentially identical to that used to prepare the tetrabutyl-Ethylenediaminetetraacetate of Example 1 was used to prepare tetramethyl-Ethylenediaminetetraacetate. In this example, $H_4$EDTA contaminated with 170 ppm of nickel and concentrated $H_2SO_4$ in a 1:10 molar ratio were refluxed in an excess of methanol to produce tetramethyl-Ethylenediaminetetraacetate. The tetramethyl-Ethylenediaminetetraacetate was recovered from the solution. The nickel content of the ester was below 0.87 ppm.

Example 4

The tetramethyl-Ethylenediaminetetraacetate of Example 3 was hydrolyzed into $H_4$EDTA in this example. The tetramethyl-Ethylenediaminetetraacetate was mixed with NaOH in a 1:10 molar ratio with an excess of water and refluxed gently while stirring for about fifteen hours. The pH of the solution was made acidic and the solid $H_4$EDTA was filtered from the acidified solution and dried in a vacuum oven at 70°C to a constant weight. A nickel analysis of the recovered $H_4$EDTA revealed the concentration to be below the detection level of 0.45 ppm.

**Claims**

1. A process for removing metal from an aminopolycarboxylic acid, said process comprising esterifying said aminopolycarboxylic acid and separating at least some of said metal from the resulting ester.

2. A process as claimed in claim 1 wherein said aminopolycarboxylic acid comprises ethylenediaminetetraacetic acid.

3. A process as claimed in either of claims 1 and 2 which comprises producing an ester-containing liquid phase and a metal-containing solid phase and separating said solid and liquid phases.

4. A process as claimed in any one of claims 1 to 3 wherein said metal is in the form of a metal salt or complex or inorganic metal compound.

5. A process as claimed in any one of claims 1 to 4 wherein esterification of said acid is effected in the presence of an organic solvent.

6. A process as claimed in claim 5 wherein esterification of said acid is effected in the presence of a non-protic organic solvent.

7. A process as claimed in claim 6 wherein said solvent comprises methanol.

8. A process as claimed in any one of claims 1 to 7 wherein esterification of said acid is effected by reaction with a primary alcohol.

9. A process as claimed in any one of claims 1 to 8 wherein esterification of said acid is effected by reaction with a $C_{1-10}$ alcohol.

10. A process as claimed in claim 9 wherein esterification of said acid is effected by reaction with a $C_{1-10}$ aliphatic alcohol.

11. A process as claimed in claim 9 wherein esterification of said acid is effected by reaction with methanol.

12. A process as claimed in claim 9 wherein esterification of said acid is effected by reaction with a $C_{6-10}$ aromatic alcohol.

13. A process as claimed in any one of the preceding claims wherein said metal comprises a radioactive metal isotope.

14. A process as claimed in any one of claims 1 to 13 wherein esterification of said acid is effected by reaction with an esterification reagent in the presence of a non-protic organic solvent at a temperature of from 0°C to the boiling point of the reaction mixture for a period of from 15 minutes to 24 hours whereby to produce a metal salt in the solid phase and an ester-containing liquid phase which are separated to yield a metal containing solid phase and a clarified ester-containing liquid phase.

15. A process as claimed in any one of claims 1 to 14 wherein said metal is separated from said ester by filtration.

16. A process as claimed in any one of claims 1 to 15 wherein said ester, after separation thereof from said metal, is incinerated.

17. A process as claimed in any one of claims 1 to 16 wherein the ester-containing component produced on separation therefrom of said metal is hydrolyzed to produce a hydrolysis product including a precipitate of said acid substantially free of said metal and a hydrolysis solution comprising non-protic organic solvent, water and alcohol.

18. A process as claimed in claim 17 wherein said precipitate is recovered from said hydrolysis solution, and said non-protic organic solvent and alcohol are recovered from the hydrolysis solution and recycled to the esterification reaction.

19. A process as claimed in any one of claims 1 to 18 wherein the ester-containing component produced on separation therefrom of said metal is distilled to yield substantially pure component streams of esterification reagent, organic solvent, alcohol and ester.

20. A process as claimed in claim 19 wherein said streams of esterification reagent, alcohol, and organic solvent are recycled for further use in the esterification reaction.

21. A process as claimed in either of claims 19 and 20 wherein said ester stream is disposed of.

22. A process as claimed in either one of claims 19 and 20 wherein said ester stream is hydrolyzed under acidic conditions to produce a precipitate of said acid substantially free of said metal.

23. A process as claimed in either one of claims 19 and 20 wherein said ester stream is hydrolyzed under basic conditions to produce a solution of a salt of said acid substantially free from said metal.

24. A process as claimed in claim 1 for removing a solid radioactive metal salt contained in an $H_4$EDTA precipitate comprising:
    (a) esterifying the solid radioactive metal salt-containing $H_4$EDTA precipitate in the presence of methanol at a temperature of from 0°C to the boiling point temperature of the reaction mixture but in any event no greater than about 220°C for a period of time of from 15 minutes to 24 hours to produce an esterification reaction mixture comprising the solid radioactive metal salt, methanol, and EDTA ester, and
    (b) filtering the esterification reaction mixture to recover the solid radioactive metal salt and to define a clarified esterification reaction mixture.

25. An aminopolycarboxylic acid perester other than tetramethyl-EDTA.

26. An EDTA-tetraester as claimed in claim 25 wherein the ester groups are of formula $COOR_1$, where each $R_1$ which may be the same or different, is an aliphatic or aromatic group.

27. An ester as claimed in claim 26 wherein each $R_1$ group contains at least two carbon atoms.

28. An ester as claimed in claim 26 wherein each $R_1$ group is a $C_{3-10}$ aliphatic group.

29. An ester as claimed in claim 26 wherein each

$R_1$ group is a $C_{6-10}$ aromatic group.

30. A homo-perester as claimed in any one of claims 25 to 29.

31. An ester as claimed in claim 24 being the tetrabutyl ester of EDTA.

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5 ) |
|---|---|---|---|
| X | US-A-4 104 252 (I. HECHENBLEIKNER & W. P. ENLOW) <br> * claim 1; examples 1,2 * <br> * see also trimethyl nitriloacetate * <br> --- | 25,30 | C07C227/40 <br> C07B63/02 <br> G21F9/06 <br> C02F5/12 <br> C07C229/16 |
| X | US-A-3 636 083 (E.D. WEIL, W. STAMM & B. MIRVISS ) <br> * claim 1; example 1 * <br> --- | 25,30 | |
| X | EP-A-0 211 572 (SUMITOMO) <br> * example 1 * <br> * Pentamethyldiethylenetriamine pentaacetate * <br> --- | 25,30 | |
| X | GB-A-1 076 146 (SINCLAIR) <br><br> * examples I-IV; table I * <br> * C4, C5 & C8 tetraalkyl EDTA esters * <br> --- | 25-28, 30,31 | |
| X | GB-A-1 453 694 (SATRA) <br> * page 3, column 1-2 * <br> --- | 25-31 | |
| X | EP-A-0 271 260 (3M) <br> * page 4 - page 5 * <br> * Formulae I-IV, IV-B, I-B, III-B * <br> * page 32; examples 15,17 * <br> --- | 25-31 | **TECHNICAL FIELDS SEARCHED (Int. Cl.5 )** <br><br> C07C <br> G21F |
| X | INORGANICA CHIMICA ACTA <br> vol. 144, no. 1, 1 April 1988, LAUSANNE CH <br> pages 81 - 87; <br> C. J. COOK & J. TOPICH: 'Synthesis and Characterisation of cis-Dioxomolybdenum( VI) Complexes' <br> * page 83, column 1, paragraph II * <br> --- | 25 | C02F <br> C22B <br> C07B |
| X | JOURNAL OF APPLIED CHEMISTRY AND BIOTECHNOLOGY. <br> vol. 22, no. 12, 1972, OXFORD GB <br> pages 1267 - 1276; <br> D. J. ALNER & CO: 'Esterification of Ethylenediaminetetraacetic Acid' <br> * page 1271 * <br> --- | 25-31 | |

The present search report has been drawn up for all claims

| Place of search | Date of completion of the search | Examiner |
|---|---|---|
| THE HAGUE | 30 JANUARY 1992 | WELLS A.G. |

CATEGORY OF CITED DOCUMENTS

X : particularly relevant if taken alone
Y : particularly relevant if combined with another
   document of the same category
A : technological background
O : non-written disclosure
P : intermediate document

T : theory or principle underlying the invention
E : earlier patent document, but published on, or
   after the filing date
D : document cited in the application
L : document cited for other reasons
........................................................................
& : member of the same patent family, corresponding
   document

EPO FORM 1503 03.82 (P0401)

European Patent
Office

EUROPEAN SEARCH REPORT

Application Number

EP    91 30 6977
Page 2

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5 ) |
|---|---|---|---|
| A | US-A-4 205 023 (J. F. ANZERBERGER)<br>* abstract *<br>--- | 1 | |
| A | WORLD PATENTS INDEX<br>Section Ch, Week 7109,<br>Derwent Publications Ltd., London, GB;<br>Class A, AN 71-16091S<br>& JP-B-46 007 937 (KURARAY YUKA) 1971<br>* online abstract *<br>----- | 1 | |
| | | | TECHNICAL FIELDS SEARCHED (Int. Cl.5 ) |

The present search report has been drawn up for all claims

| Place of search | Date of completion of the search | Examiner |
|---|---|---|
| THE HAGUE | 30 JANUARY 1992 | WELLS A.G. |

CATEGORY OF CITED DOCUMENTS

X : particularly relevant if taken alone
Y : particularly relevant if combined with another
   document of the same category
A : technological background
O : non-written disclosure
P : intermediate document

T : theory or principle underlying the invention
E : earlier patent document, but published on, or
   after the filing date
D : document cited in the application
L : document cited for other reasons

& : member of the same patent family, corresponding
   document

EPO FORM 1503 03.82 (P0401)